# EUROPÄISCHE PATENTANMELDUNG

(11) **EP 2 526 988 A1**
(43) Veröffentlichungstag der Anmeldung: **28.11.2012**
(21) Anmeldenummer: 12175500.3
(22) Anmeldetag: 02.11.2010
(51) Int. Cl.: A61M 13/00, A61F 9/007

(54) **Spritze**

(30) Priorität: 10.11.2009 DE 102009052552
(62) Teilanmeldung aus: 10014210.8
(71) Anmelder: Fluoron GmbH, 89077 Ulm (DE)
(72) Erfinder: Lingenfelder, Christian, 89081 Ulm (DE); Knopp, Benjamin, 89134 Blaustein (DE)
(74) Vertreter: Weinzierl, Gerhard

(57) **Zusammenfassung**

Die Erfindung betrifft eine Spritze (1) mit einem Spritzenkörper (2), der einen Innenraum (3) und eine mit dem Innenraum (3) verbundene Spitze (4) aufweist. Eine Kolbenstange (5) ist längsverschieblich im Innenraum (3) geführt. Auf der Spitze (4) ist ein Spritzenvorsatzfilter (6) fixierbar und ein Verschlussstopfen (7) kann auf dem Spritzenvorsatzfilter (6) befestigt werden.

## Beschreibung

### Gebiet der Erfindung

Die Erfindung betrifft eine Spritze gemäß dem Oberbegriff des Anspruches 1.

Insbesondere betrifft die Erfindung ein Ready to Use-System einer Gastamponade zur Anwendung in der Medizin. Die Tamponade wird zum Auffüllen einer natürlichen, z.B. durch eine alterungsbedingte Glaskörperschrumpfung, oder künstlichen, z.B. durch eine Vitrektomie entstandenen Kavität verwendet.

### Hintergrund der Erfindung

Bedingt durch die steigende Lebenserwartung des Menschen nehmen alterungsbedingte Erkrankungen wie Netzhautveränderungen bzw. Netzhautablösungen, grüner Star, grauer Star, sowie alterungsbedingte Makuladegeneration und diabetesbedingte Retinopathie zu. Um diese und auch weitere Erkrankungen des Auges zu behandeln, ist meistens eine Vitrektomie (Entfernung des Glaskörpers) notwendig. Der entstandene Hohlraum muss wieder aufgefüllt werden, um ein Zusammenfallen des Glaskörperraumes zu verhindern. Zu diesem Zweck werden "schwere Gase" wie SF₆, C₂F₆ oder C₃F₈ verwendet. Von schweren Gasen spricht man allgemein, wenn die Gase eine deutlich höhere Dichte im Vergleich zu normaler Raumluft besitzen.

Die therapeutische Wirkung entsteht in den meisten Fällen nicht durch das Gas selbst, sondern vielmehr durch die Gas-Flüssigkeitsgrenzfläche. Diese Oberflächenspannung beugt dem Durchtritt von Gas durch ein Netzhautloch in den Subretinalraum vor, zusätzlich wird das Netzhautloch ausgespannt und ein weiteres Durchtreten von Flüssigkeit in den Subretinalraum verhindert.

Nach der Injektion des schweren Gases beginnt eine Diffusion von O₂ und CO₂ aus dem Blut ins Auge, wodurch das Volumen der Gasblase zunimmt. Nach einigen Stunden stellt sich ein Diffusionsgleichgewicht für O₂ und CO₂ ein, für N₂ jedoch erst nach einigen Tagen.

Die schweren Gase werden transretinal über die Aderhaut aufgenommen und verlassen je nach Typ das Auge innerhalb von 1 bis 2 Wochen.

Die in der Chirurgie verwendeten Gase werden in den meisten Fällen direkt während der Operation aus einer Stahlflasche in das zu verwendende Medium überführt.

Das US-Patent 6,866,142 B2 beschreibt ein System, welches aus einer bereits gasgefüllten Einmalspritze besteht, die zur besseren Gasdichtigkeit in einem Behälter aufbewahrt wird, der mit dem gleichen Gas wie die Einmalspritze gefüllt ist.

Alle oben aufgeführten Systeme haben mehrere Nachteile.
- Bei allen Systemen werden zum Teil erhebliche Mengen an SF₆, C₂F₆ und C₃F₈ in die Umwelt entlassen, obwohl diese Gase zu den stärksten bekannten Treibhausgasen zählen; so hat z.B. 1 kg SF₆ die gleichen Auswirkungen wie 22,2 t CO₂ [Verordnung EG Nr. 842/2006].
- Die Abfüllung von Gastamponaden aus der Stahlflasche im OP ist nicht zugelassen, da Stahlflaschen in einem speziellen Stahlschrank für Druckbehälter aufbewahrt werden müssen.
- Die auf dem Markt befindlichen Einmaldosen müssen in mehreren Schritten vorbereitet werden, bevor sie vom Chirurgen verwendet werden können.

Es ist daher Aufgabe der vorliegenden Erfindung, eine Spritze der im Oberbegriff des Anspruchs 1 angegebenen Art zu schaffen, die dazu in der Lage ist, die zuvor erläuterten Nachteile des Standes der Technik auszuräumen und insbesondere einfach aufzubewahren und zu bedienen ist.

Die Lösung dieser Aufgabe erfolgt durch die Merkmale des Anspruchs 1.

Die Unteransprüche haben vorteilhafte Weiterbildungen der Erfindung zum Inhalt.

### Detaillierte Beschreibung der Erfindung

Somit betrifft die Erfindung ein System mit einer Einmalspritze, vorzugsweise 50 ml, mit Luer-Lock-Ansatz, welche als Gasbehälter dient, mit einem auf die Spritze aufgeschraubten Spritzenvorsatzfilter mit beiderseitigem Luer-Lock-Anschluss und mit einem Luer-Stopfen, um das System zu verschließen.

Das montierte System (Einmalspritze und Spritzenvorsatzfilter) wird mit wenigen Millilltern des jeweiligen Gases gefüllt und mit dem Luer-Stopfen verschlossen.

Das System wird vorzugsweise mit einem Aufkleber versehen, welcher die Mischungsverhältnisse für SF₆ (20% Gas/80% Luft), C₂F₆ (16% Gas/84% Luft) und C₃F₈ (12% Gas/88% Luft) farblich unterscheidet, sowie den Maximumwert kennzeichnet, bis zu dem der Kolben herausgezogen werden muss/darf, um das Mischungsverhältnis einzustellen. Das verschlossene System wird verpackt, vorzugsweise in einen Sterilbeutel, und anschließend sterilisiert, vorzugsweise in einem Dampfautoklaven.

Weitere Einzelheiten, Merkmale und Vorteile der Erfindung ergeben sich aus nachfolgender Beschreibung von Ausführungsbeispielen anhand der Zeichnung.

Darin zeigt:
Fig. 1 eine schematisch leicht vereinfachte Darstellung einer ersten Ausführungsform einer erfindungsgemäßen Spritze, und
Fig. 2 eine der Fig. 1 entsprechende Darstellung einer zweiten Ausführungsform der erfindungsgemäßen Spritze.

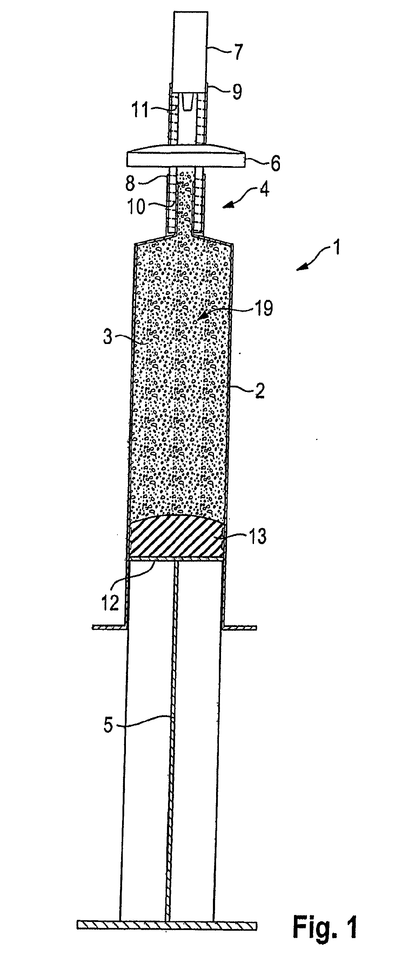

In Fig. 1 ist eine erste Ausführungsform einer erfindungsgemäßen Spritze 1 dargestellt, die einen zylinderförmigen Spritzenkörper 2 aufweist, der einen Innenraum 3 umfasst.

Der Innenraum 3 ist mit einer Spitze 4 verbunden und im Innenraum 3 ist eine Kolbenstange 5 längsverschieblich geführt.

Wie Fig. 1 verdeutlicht, ist auf der Spitze 4 ein Spritzenvorsatzfilter 6 fixiert. Das gesamte System ist ferner mit einem Verschlussstopfen 7 gasdicht verschlossen, der wiederum auf dem äußeren Ende des Spritzenvorsatzfilters 6 fixierbar ist.

Bei einer besonders bevorzugten Ausführungsform weist der Spritzenvorsatzfilter 6 einen ersten Fixierungsabschnitt 8 zur Anbringung an der Spitze 4 und einen zweiten Fixierungsabschnitt 9 zur Festlegung des Verschlussstopfens 7 auf.

Vorzugsweise sind der Spritzenvorsatzfilter 6 und der Verschlussstopfen 7 mittels jeweils einer Schraubverbindung 10 bzw. 11 fixierbar. Bei einer besonders bevorzugten Ausführungsform sind die Schraubverbindungen 10 und 11 jeweils als Luer-Lock-Verschluss ausgebildet.

Wie Fig. 1 ferner verdeutlicht, weist die Kolbenstange 5 an ihrem im Innenraum 3 angeordneten Endbereich 12 einen Gummistopfen 13 auf, der vorzugsweise siliconisiert ist.

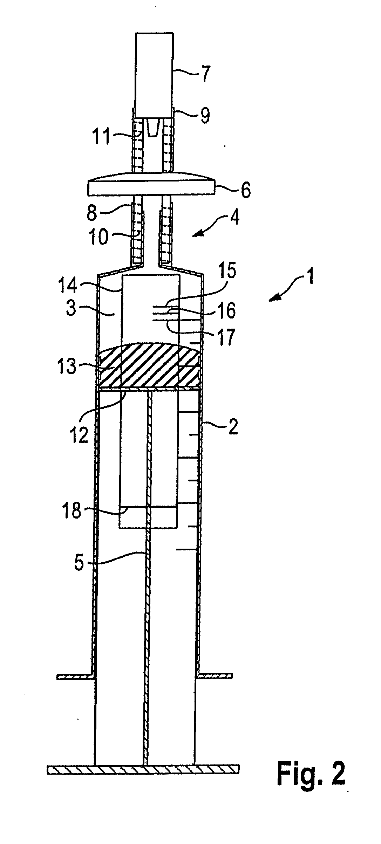

Die Ausführungsform gemäß Fig. 2 entspricht weitestgehend derjenigen der Fig. 1, so dass alle einander entsprechenden Merkmale mit denselben Bezugsziffern wie in Fig. 1 versehen sind. Insoweit kann auf die voranstehende Beschreibung der Fig. 1 verwiesen werden.

Die Ausführungsform der Spritze 1 gemäß Fig. 2 zeichnet sich dadurch aus, dass auf dem Spritzenkörper 2 ein Etikett 14 fixiert ist. Wie Fig. 2 verdeutlicht, weist das Etikett 14 Markierungen 15, 16 und 17 für unterschiedliche Mischungsverhältnisse zwischen schwerem Gas 19 und der anzusaugenden Luft auf. Ferner ist eine Markierung 18 für den maximalen Kolbenhub vorgesehen.

Wie eingangs bereits erläutert, kann die Spritze 1 als Einmalspritze ausgebildet sein.

Bevorzugterweise ist das im Innenraum 3 des Spritzenkörpers 2 eingebrachte Gas ein schweres Gas insbesondere SF₆, C₃F₈ oder C₂F₆.

Neben der voranstehenden schriftlichen Offenbarung der Erfindung wird hiermit explizit auf deren zeichnerische Darstellung in den Figuren 1 und 2 verwiesen.

### Bezugszeichenliste

- 1: Spritze
- 2: Spritzenkörper
- 3: Innenraum
- 4: Spitze
- 5: Kolbenstange
- 6: Spritzenvorsatzfilter
- 7: Verschlussstopfen
- 8: Fixierungsabschnitt
- 9: Fixierungsabschnitt
- 10, 11: Schraubverbindungen
- 13: Gummistopfen
- 14: Etikett
- 15, 16, 17: Markierung
- 19: Gas

## Patentansprüche

1. Spritze (1),
- mit einem Spritzenkörper (2),
• der einen Innenraum (3) und
• der eine mit dem Innenraum (3) verbundene Spitze (4) aufweist;
- mit einer Kolbenstange (5), die längsverschieblich im Innenraum (3) geführt ist, **dadurch gekennzeichnet,**
- **dass** ein Spritzenvorsatzfilter (6) auf der Spitze (4) fixierbar ist, und
- **dass** ein Verschlussstopfen (7) auf dem Spritzenvorsatzfilter (6) fixierbar ist.

2. Spritze nach Anspruch 1, **dadurch gekennzeichnet, dass** der Spritzenvorsatzfilter (6) einen ersten Fixierungsabschnitt (8) zur Anbringung an der Spitze (4) und einen zweiten Fixierungsabschnitt (9) zur Festlegung des Verschlussstopfens (7) aufweist.

3. Spritze nach Anspruch 1 oder 2, **dadurch gekennzeichnet, dass** der Spritzenvorsatzfilter (6) und der Verschlussstopfen (7) mittels jeweils einer Schraubverbindung (10 bzw. 11) fixierbar ist.

4. Spritze nach Anspruch 3, **dadurch gekennzeichnet, dass** die Schraubverbindung (10 bzw. 11) als Luer-Lock-Verschluss ausgebildet ist.

5. Spritze nach einem der Ansprüche 1 bis 4, **dadurch gekennzeichnet, dass** die Kolbenstange (5) an ihrem im Innenraum (3) angeordneten Endbereich (12) mit einem Gummistopfen (13) versehen ist.

6. Spritze nach Anspruch 5, **dadurch gekennzeichnet, dass** der Gummistopfen (13) siliconisiert ist.

7. Spritze nach einem der Ansprüche 1 bis 6, **dadurch gekennzeichnet, dass** auf dem Spritzenkörper (2) ein Etikett (14) mit Mischungsverhältnismarkierungen (15, 16 bzw. 17) und einer Markierung (18) für den maximalen Kolbenhub anbringbar ist.

8. Spritze nach einem der Ansprüche 1 bis 7, **dadurch gekennzeichnet, dass** sie als Einmalspritze ausgebildet ist.

9. Spritze nach einem der Ansprüche 1 bis 8, **dadurch gekennzeichnet, dass** der Spritzenkörper (2) mit einem schweren Gas (19) befüllbar ist.

10. Spritze nach Anspruch 9, **dadurch gekennzeichnet, dass** das schwere Gas **SF₆,** C₃F₈ oder C₂F₆ ist.
